# EUROPEAN PATENT SPECIFICATION

(11) **EP 2 847 163 B1**
(45) Date of publication and mention of the grant of the patent: **21.02.2018**
(21) Application number: 13787229.7
(22) Date of filing: 14.03.2013
(51) Int. Cl.: C07C 337/00, C07C 337/08, A61P 35/00, C07D 213/53

(54) **IRON CHELATORS AS HIV-1 INHIBITORS**
EISENCHELATOREN ALS HIV-1-INHIBITOREN
CHÉLATEURS DU FER EN TANT QU'INHIBITEURS DU VIH-1

(30) Priority: 09.05.2012 US 201261644842 P
(43) Date of publication of application: 18.03.2015
(73) Proprietor: Howard University, Washington, DC 20059 (US)
(72) Inventor: NEKHAI, Sergei, McLean, VA 22101 (US); KOVALSKYY, Dmytro Borisovych, San Antonio, TX 78231 (US)
(74) Representative: Glansdorp, Freija Gwendolyn
(86) International application number: PCT/US2013/031231
(87) International publication number: WO 2013/169361

(56) References cited:
- WO-A1-2010/000008
- WO-A1-2010/147621
- US-A1- 2009 286 799
- CASE F H ET AL: "preparation and chromogenic properties of semicarbazones containing the ferroin group", JOURNAL OF CHEMICAL AND ENGINEERING DATA, AMERICAN CHEMICAL SOCIETY, US, vol. 25, no. 4, 1 January 1980 (1980-01-01), pages 404-405, XP002436212, ISSN: 0021-9568, DOI: 10.1021/JE60087A029
- W. HAMPEL: "Reaktionen mit Diazoketonen. IX. Addition von Diazoketonen an ?-Dicarbonylverbindungen", JOURNAL FUER PRAKTISCHE CHEMIE, vol. 311, no. 6, 1 January 1969 (1969-01-01), pages 1058-1064, XP55233859, DE ISSN: 0021-8383, DOI: 10.1002/prac.19693110624
- Z. DEBEBE ET AL: "Iron Chelators of the Di-2-pyridylketone Thiosemicarbazone and 2-Benzoylpyridine Thiosemicarbazone Series Inhibit HIV-1 Transcription: Identification of Novel Cellular Targets--Iron, Cyclin-Dependent Kinase (CDK) 2, and CDK9", MOLECULAR PHARMACOLOGY, vol. 79, no. 1, 18 October 2010 (2010-10-18), pages 185-196, XP055176960, ISSN: 0026-895X, DOI: 10.1124/mol.110.069062
- DEBEBE ET AL.: 'Iron Chelators of the Di-2-pyridylketone Thiosemicarbazone and 2- Benzoylpyridine Thiosemicarbazone Series Inhibit HIV-1 Transcription: Identification of Novel Cellular Targets-Iron, Cyclin-Dependent Kinase (CDK) 2, and CDK9' MOLECULAR PHARMACOLOGY vol. 79, no. 1, 2011, pages 185 - 196, XP055176960
- KALINOWSKI ET AL.: ''The Evolution of Iron Chelators for the Treatment of Iron Overload Disease and Cancer' PHARMACOLOGICAL REVIEWS vol. 57, no. 4, 2005, pages 547 - 583, XP002473786

## Description

This PCT application claims the filing date benefit of U.S. Provisional Application 61/644,842, filed May 9, 2012.

This work was made with U.S. Government support under the Civilian Research and Development Foundation grant UKB2-2927-KV-07, (CRDF is an independent nonprofit organization that promotes international scientific and technical collaboration through grants, technical resources, and training. CRDF is based in Arlington, Virginia); NIH NHLBI grant R25 HL003679-08, NIH NIGMS grant 1SC1GM082325-01 and NCRR grant RCMI-NIH 2G12RR003048, with the last three awarded by the Federal Agency "National Institutes of Health." The government has certain rights in this work.

### Technical Field

The present invention relates to novel metal ion chelators, including chelators for chelating iron, including iron (II) and iron (III) cations, and, in particular, (thio) semicarbazone compounds, as well as said compounds for use in therapeutic uses. The novel metal ion chelators include representative PpYaT and PpYeT compounds.

### Background

Iron (Fe) is fundamentally involved in many important HIV-1 processes such as reverse transcription, viral gene expression and capsid assembly (Drakesmith et al., 2008). Increased iron stores are correlated with faster HIV-1 progression in HIV-positive patients.

Desferrioxamine (DFO) is an iron chelator currently used for the treatment of iron overload disease. However, its use as an anti-cancer or anti-viral agent is disadvantageously severely handicapped, e.g. rather limited, by its modest anti-proliferative activity, short half-life and high cytoxicity. Another effort involves a recently approved iron chelator ICL670 (Exjade) for the treatment of iron overload (Porter, 2006). However, ICL670 suffers from disadvantages, including the drawback of that relatively high concentrations of ICL670 are required in order to exhibit inhibition of HIV-1 replication. These drawbacks and other disadvantages in the art have tended to discourage development of chelators into a clinically useful anti-HIV-1 thereapeutic agents.

In the aftermath of the presently encountered discouraging results, the need for new therapeutically effective approaches for inhibiting HIV-1 replication remains unfulfilled, and developing efficacious chelators specific to inhibiting the transcription of viral Tat, CDK2, CDK9/cyclinT1 and NFkB remains elusive.

### Summary

The present approach for inhibiting HIV replication relates to novel chelators, including novel iron chelators, which inhibit HIV-1 proliferation by chelating intracellular Fe and interfering with the activity of host cell cycle-dependent kinase-2 (CDK2) and cell cycle-dependent kinase-9 (CDK2) required for HIV-1 proliferation. In addition, the iron chelators induce expression of NF-kB inhibitor, IKBα, and may inhibit HIV-1 transcription by suppressing the NF-kB activity. Also the iron chelators induce expression of heme oxygenase-1 (HO-1) which was previously shown to inhibit HIV-1 Devadas et al., (2006 & 2010) and which expression may also contribute to the anti-HIV-1 effect of the iron chelators. These novel iron chelators have anti-viral activity, which may synergistically target multiple biochemical pathways associated with HIV-1 replication, with fewer side effects.

Host cell proteins that activate HIV-1 transcription are dependent on the presence of metal ions and the concentration thereof, in particular, iron (II) and iron (III) cations, and represent an HIV-1 specific target for intracellular iron chelation to inhibit HIV-1 transcription and replication. The present invention is solely defined by the appended claims.

In an aspect of the disclosure, a metal ion chelator is represented by formula 1 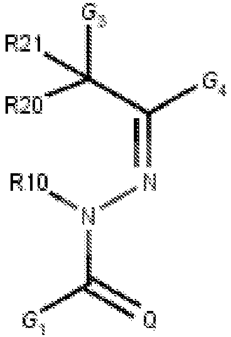 wherein
G₁ is NR₂R₃ or CR₂R₃R₄,
R₂, and R₃, can be the same or different and can be individually selected from hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
R₄ is selected from the group consisting of hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
R₁₀ can be hydrogen or a group that does not hinder metal ion chelation, including a hydrocarbyl group, a substituted hydrocarbyl group, aryl group, substituted aryl group, a heteroatom containing group, substituted heteroatom containing group, as examples, with hydrogen being presently preferred;
R₂₀ and R₂₁ can be the same or different and are selected from the group consisting of hydrogen, halogen, optionally substituted alkyl, optionally substituted alkenyl, optionally substituted alkynyl, optionally substituted amino, optionally substituted heteroaromatic, an optionally substituted bicyclic group, and an optionally substituted aromatic group;
G₃ may be selected from an optionally substituted aromatic ring, such as an optionally substituted 5- or 6-membered aromatic ring, an optionally substituted heteroaromatic ring, such as a 5- or 6-membered heteroaromatic ring, an optionally substituted bicyclic group, an optionally substituted aromatic group, an optionally substituted alkyl group, optionally substituted cycloalkyl group, or an optionally substituted heterocycloalkyl group, provided that when G3 is an optionally substituted alkyl group, R₂₀ and are not both hydrogen nor is one hydrogen while the other is alkyl;
G4 is an N-heteroaryl linked to the core structure at the 2-position (ortho to the nitrogen atom), and
Q is S or O, or
   a pharmaceutically acceptable salt thereof.

For example, in one aspect, in a compound according to formula 1, G₃ may be selected from an optionally substituted phenyl, naphthyl, pyridyl, pyrimidinyl, triazinyl, oxazolyl, pyrrolyl, and furanyl group. Optional substituents include electron withdrawing groups and electron donating groups. Examples of optional substituents include alkyl, alkenyl, alkynyl, C₆₋₁₀ aryl, halo, amino, hydroxyl, O-alkyl, S-alkyl, nitro, cyano, C(O)-alkyl, C(O)--O-alkyl, C(O)--NH(alkyl), and C(O)--N(alkyl)₂.

In another of its aspects, an ion chelator is represented by formula 2 below, 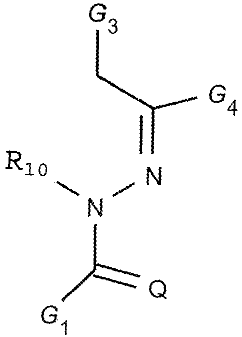 wherein
Q is O or S;
G₁ is as described for formula 1;
R₁₀ is as described for formula 1;
G₄ is as described in formula 1; and
G₃ is as described as in formula 1, or
a pharmaceutically acceptable salt thereof.

An ion chelator may preferably be the sulfur analog of the compound represented formula 1, in which case Q is S.

In another of its aspects, a metal ion chelator comprises a compound represented by formula 3: 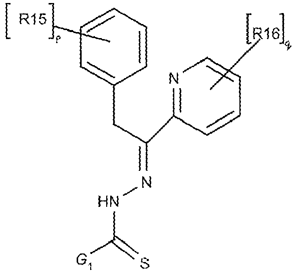 wherein
Q is S;
each R₁₅ is independently selected from halogen, alkyl, alkenyl, optionally substituted amino, hydroxyl, --O-alkyl, --S-alkyl, --C(O)-alkyl, --C(O)-alkenyl, --C(O)--O-alkyl, nitro and cyano,
p is 0, 1, 2, 3 or 4;
each R₁₆ is is independently selected from halogen, alkyl, alkenyl, optionally substituted amino, hydroxyl, --O-alkyl, --S-alkyl, --C(O)-alkyl, --C(O)-alkenyl, --C(O)--O-alkyl, nitro and cyano,
q is 0, 1, 2, 3 or 4; and
G₁ is NR₂R₃ or CR₂R₃R₄ wherein R₂ and R₃ and R₄ are as described herein formula 1, or a pharmaceutically acceptable salt thereof.

In another of its aspects, a metal ion chelator comprises a compound represented by formula 4: 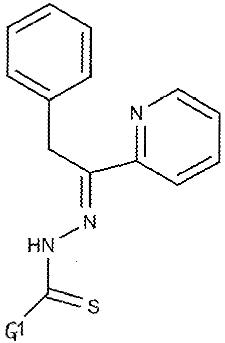 wherein G₁ is NR₂R₃ or CR₂R₃R₄ wherein R₂ and R₃ and R₄ are as described herein formula 1, or a pharmaceutically acceptable salt thereof.

In another of its aspects, a metal ion chelator comprises a compound represented by formula 5: 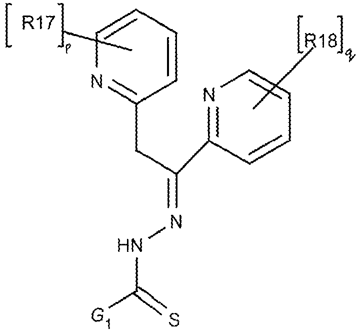
wherein G₁ is NR₂R₃ or CR₂R₃R₄ wherein R₂ and R₃ and R₄ are as described herein formula 1;
each R₁₇ is independently selected from halogen, alkyl, alkenyl, optionally substituted amino, hydroxyl, --O-alkyl, --S-alkyl, --C(O)-alkyl, --C(O)-alkenyl, --C(O)--O-alkyl, nitro and cyano;
p is 0, 1, 2, 3 or 4;
each R₁₈ is independently selected from halogen, alkyl, alkenyl, optionally substituted amino, hydroxyl, --O-alkyl, --S-alkyl, --C(O)-alkyl, --C(O)-alkenyl, --C(O)--O-alkyl, nitro and cyano; and
q is 0, 1, 2, 3 or 4, or
a pharmaceutically acceptable salt thereof.

According to an aspect of the invention, a metal ion chelator comprises a compound represented by the formula 6: 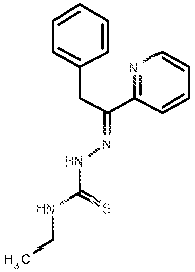 PpYeT, or a pharmaceutically acceptable salt thereof.

According to an aspect of the invention, a metal ion chelator comprises a compound represented by the formula 7: 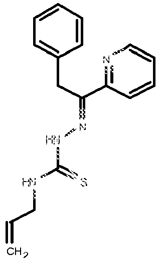 PpYaT, or a pharmaceutically acceptable salt thereof.

In another aspect of the disclosure, instead of Q being =S as in formulas 3-7, Q can be =O.

G₃ is bonded via -(CR₂₀R₂₁)- to the core of the compound as shown in formulas 1-7. In another aspect, it will be appreciated that G₃ may be bonded via another group, such as -(CH₂)_{g}(CR₂₀R₂₁)- wherein g is 1, 2 or 3, to the core of the compound, and G3 is preferably not optionally substituted alkyl when R₂₀ and are hydrogen or when one is hydrogen and the other is alkyl. In another aspect, it will be appreciated that in a compound according to formulas 1-7, in principle, G₄ may be bonded through its 2-position to another group, such as -(CH₂)ₕ(CR₂₀R₂₁)- wherein h is 0, 1, 2 or 3, to the core of the chelator compound.

An aspect of the disclosure includes a metal ion complex that comprises a transition metal ion complex, such as copper, iron, zinc, palladium, platinum, or gallium ion complex of any of the compounds of the present invention. The metal ion may have a lower or a higher valence. In one embodiment, the metal ion is iron (II). In another embodiment, the metal ion is iron (III). In a further embodiment, the metal ion is Cu (I) or Cu (II), as examples.

Suitable metal ions to be chelated include transition metal ions. For example, the metal ions can be selected from Periods 4 and 5 of the Periodic Table, and exemplary metal ions include iron (II), iron (III), copper (II), zinc (II), palladium (II), platinum (II), or gallium (III), as examples. Iron ion(s) are preferred.

In another aspect, any of the metal ion chelators functions as a tridentate ligand(s). In an aspect of the disclosure at least two of the metal ion chelators form a hexa-coordinate complex with a metal ion, for example, iron (II) or iron (III).

In another aspect, a pharmaceutical composition comprises a pharmaceutically acceptable diluent, adjuvant or carrier together with at least one of the metal ion chelators as described herein.

A pharmaceutical composition comprises at least one compound of formula 1 or another ion chelator compound described herein.

Compounds as defined herein, and compositions thereof, can be used for in vitro or in vivo metal ion chelation. For example, in one embodiment, compounds represented by any of the formulas, preferably representative PpYeT and PpYaT compounds, as defined herein, and compositions thereof are compounds for use for chelating cellular iron.

Compounds represented by the formulas, and compositions thereof, are suitable for HIV-1 therapy or treating HIV-1 replication. Compounds represented by any of the formulas, preferably representative PpYeT and PpYaT compounds, and compositions thereof are useful for modifying cellular function of at least one of CDK2, CDK9 and Tat required for HIV-1 transcription.

Compounds represented by the formulas, preferably representative PpYeT and PpYaT compounds, and compositions thereof are useful for inhibiting cellular HIV-1 proliferation and/or inhibiting HIV-1 replication in HIV-1 viral infected cells. Thus, a method for inhibiting HIV-1 replication or proliferation comprises administering a compound represented by any of the formulas, preferably representative PpYeT and PpYaT compounds, to HIV-1 viral infected cells. In another aspect a method for inhibiting HIV-1 replication or proliferation comprises introducing an ion chelator compound of the present invention, such as one represented by any of formulas 1 through 7, into a target infected cell.

Compounds represented by formulas, preferably representative PpYeT and PpYaT compounds, are inhibitory for HIV-1 replication and can be less cytotoxic as compared to other iron chelators. These relatively smaller molecules decrease the activity of host cell CDK2, CDK9 and viral Tat. In one aspect, compounds described above induce expression of NF-kB inhibitor, IKBα, and HO-1. While expression of these genes may be known to interfere with the replication of HIV-1, inducing expression of NF-kB inhibitor, IKBα, and HO-1 with an ion chelator compound of the invention is novel. In one aspect, an iron chelator compound(s) described herein can be used for the treatment of HIV-1 infections by inhibiting HIV-1 transcription, including inhibition of NF-kB through the increased expression of IkB. For example, a low nanomolar IC₅₀ for an iron chelator, such as those exemplified by formulas 5-7, and their lack of toxicity, demonstrates usefulness as anti-HIV-1 therapeutics.

Compounds of the present invention exhibit efficiency in chelating a metal cation (e.g., iron) in combating HIV. The compounds of the present invention exhibit efficiency as metal cation chelators that is at least as potent as a compound known as Bp4eT as seen from data obtained from representative compounds. The compounds of the present invention are surprisingly less toxic than compounds known as Bp4eT and Bp4aTas seen from data from representative compounds. These advantages of the present compounds were unexpected given the characteristics and structure of the Bp4eT and Bp4aT compounds.

### Brief Description Of The Drawings

The foregoing features, as well as other features, will become apparent with reference to the description and Figures below, and in which:
Figure 1 illustrates activation of HIV-1 transcription by Tat;
Figure 2 depicts line graphs of the effect of iron chelators on one round of HIV-1-Luc replication in CEM cells;
Figure 3 depicts line graphs of the toxicity of iron chelators in CEM cells;
Figure 4 depicts line graphs of the PpY Iron chelators efficiently chelating cellular iron;
Figure 5 depicts bar graphs of iron chelators inducing expression of IkB;
Figure 6 depicts bar graphs of iron chelators inducing expression of heme oxygenase-1 (HO-1);
Figure 7 depicts graphically data regarding toxicity of iron chelators in CEM T cells over the course of a 72 hours treatment; and
Figure 8 depicts graphically data regarding the effects of iron chelators on one round of HIV-1-Luc replication in CEM cells.

### Detailed Description of Preferred Embodiments

The term "alkyl group" includes straight chain or branched chain saturated aliphatic groups having from 1 to 10 carbon atoms, for example, 1, 2, 3, 4, 5, 6, 7, 8, 9 or 10 carbon atoms, or cyclic saturated aliphatic groups having from 3 to 8 carbon atoms, for example, 3, 4, 5, 6, 7, or 8 carbon atoms, or bicyclic saturated aliphatic groups having 7, 8, 9 or 10 carbon atoms. For example, the term alkyl includes, but is not limited to, methyl, ethyl, 1-propyl, isopropyl, 1-butyl, 2-butyl, tert-butyl, amyl, 1,2-dimethylpropyl, 1,1-dimethylpropyl, pentyl, isopentyl, hexyl, 4-methylpentyl, 1-methylpentyl, 2-methylpentyl, 3-methylpentyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, 2-ethylpentyl, 3-ethylpentyl, heptyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3-trimethylbutyl, 1,1,2-trimethylbutyl, 1,1,3-trimethylbutyl, 5-methylheptyl, 1-methylheptyl, octyl, nonyl, decyl, cyclopropyl, 2-methylcyclopropyl, cyclobutyl, cyclopentyl, 2-methylcyclopentyl, 3-methylcyclopentyl, cyclohexyl, and the like. Optionally substituted lower alkyl includes C₁ to C₄ alkyl. The substituent(s) can be halogen, sulfur and oxygen, as examples.

Herein the term "alkenyl" (or alkenyl group) means straight or branched chain unsaturated aliphatic hydrocarbon groups having from 2 to 8 carbon atoms, such as, 2, 3, 4, 5, 6, 7, or 8 carbon atoms, or cyclic unsaturated aliphatic hydrocarbon groups having from 3 to 8 carbon atoms, such as, 3, 4, 5, 6, 7 or 8 carbon atoms, and combinations thereof, having at least one double bond, of either E or Z stereochemistry where applicable. Exemplary alkenyl groups include but are not limited to ethenyl, vinyl, allyl, 1-methylvinyl, 1-propenyl, 2-propenyl, 2-methyl-1-propenyl, 2-methyl-1-propenyl, 1-butenyl, 2-butenyl, 3-butentyl, 1,3-butadienyl, 1-pentenyl, 2-pententyl, 3-pentenyl, 4-pentenyl, 1,3-pentadienyl, 2,4-pentadienyl, 1,4-pentadienyl, 3-methyl-2-butenyl, 1-hexenyl, 2-hexenyl, 3-hexenyl, 1,3-hexadienyl, 1,4-hexadienyl, 2-methylpentenyl, 1-heptenyl, 2-heptentyl, 3-heptenyl, 1-octenyl, and the like. Optionally substituted lower alkenyl includes C₂ to C₄ alkenyl as examples. The substituents can be halogen, sulfur and oxygen as examples.

Herein the term "alkynyl" (or alkynyl group) means straight or branched chain unsaturated aliphatic hydrocarbon groups having from 2 to 8 carbon atoms, such as, 2, 3, 4, 5, 6, 7, or 8 carbon atoms, and having at least one triple bond. Exemplary alkynyl groups include but are not limited to ethynyl, 1-propynyl, 1-butynyl, 2-butynyl, 1-methyl-2-butynyl, 3-methyl-1-butynyl, 1-pentynyl, 1-hexynyl, methylpentynyl, 1-heptynyl, 2-heptynyl, 1-octynyl, 2-octynyl, and the like. A lower alkynyl group includes C₂ to C₄ alkynyl.

Herein "amino group", or variants thereof, such as "amino," refers to groups of the form --NR₆R₇ wherein R₆ and R₇ are individually selected and include, but not limited to, hydrogen, optionally substituted alkyl, optionally substituted alkenyl, optionally substituted alkynyl, and optionally substituted aryl groups.

Herein the term aromatic group ("aryl" or other variants) means a single, polynuclear, conjugated and fused residues of aromatic hydrocarbons having from 6 to 14 carbon atoms, such as 6, 7, 8, 9, 10, 11, 12, 13, or 14 carbon atoms. Examples of such groups include phenyl, tolyl, biphenyl, naphthyl, phenanthryl, and the like.

Herein the term halogen (halo, halide etc.) includes bromo, chloro, fluoro and iodo.

Herein the term hetero atom includes nitrogen, sulfur and oxygen.

Herein "heteroaromatic group" ("heteroaryl" and the like) include any 5-16-membered, such as,, 5-, 6-, 7-, 8-, 9-, 10-, 11-, 12-, 13-, 14-, 15- or 16-membered monocyclic, bicyclic or tricyclic aromatic group having 1, 2, 3 or 4 heteroatoms. Examples include, but are not limited to pyrrolyl, pyridyl, phenanthryl, quinolyl, isoquinolyl, imidazolyl, thiophenyl, furanyl, purinyl, indolyl, isoindolyl, phenanthridinyl, pyrimidinyl, pyridazinyl, pyrazinyl, pyrazolyl, oxazolyl, isoxazolyl, thiazolyl, benzofuranyl, and the like.

Herein "heterocycloalkyl group" or the like includes a 3-10-membered saturated or unsaturated monocyclic or bicyclic ring system, having 1, 2 or 3 heteroatoms. Such groups include piperidinyl, pyrrolidinyl, tetrahydrofuranyl, tetrahydropyrrolyl, morpholinyl, and tetrahydrothienyl, as examples.

Herein the expression "optionally substituted" means the group to which this term refers may be unsubstituted, or substituted with one or more groups independently selected from halogen, alkyl, alkenyl, alkynyl, cycloalkyl, cycloalkenyl, heterocycloalkyl, aryl, heteroaryl, cyano, cyanate, isocyanate, --O-alkyl, --S-alkyl, nitro, amino, --C(O)NH₂, --C(O)NH(alkyl), --C(O)N(alkyl)₂, --C(O)-alkyl, --C(O)-alkenyl, and -C(O)-O-alkyl, by way of examples.

Herein in formulas 1 through 7, the moiety designated or corresponding to G₄ is an N-heteroaryl linked to the core structure at the 2-position.

In one aspect of the disclosure, G3 is represented by the formula: 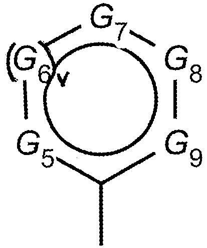
wherein each of G₅, G₆, G₇, G₈ and G₉ is independently selected from CR₁₁, NR₁₂, S and O, wherein the total number of heteroatoms in a G₃ is 1, 2 or 3;
v is 0, 1 or 2;
R₁₁ is independently selected from hydrogen, halogen, alkyl, alkenyl, optionally substituted amino, hydroxyl, --O-alkyl, --S-alkyl, --C(O)-alkyl, --C(O)-alkenyl, --C(O)-O-alkyl, nitro and cyano; and
R₁₂ is independently selected from hydrogen, halogen, alkyl, alkenyl, hydroxyl,O-alkyl, --S-alkyl, --C(O)-alkyl, and --C(O)-alkenyl, benzyl, benzylcarbamate.

In one aspect of the disclosure, G₄ is represented by the formula: 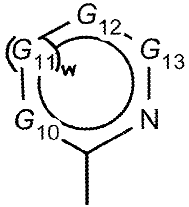
wherein each of G₁₀, G₁₁, G₁₂, and G₁₃ is independently selected from CR₁₁, NR₁₂, S and O, wherein the total number of heteroatoms in a G₄ is 1, 2 or 3;
w is 0, 1 or 2;
R₁₁ is independently selected from hydrogen, halogen, alkyl, alkenyl, optionally substituted amino, hydroxyl, --O-alkyl, --S-alkyl, --C(O)-alkyl, --C(O)-alkenyl, --C(O)-O-alkyl, nitro and cyano; and
R₁₂ is independently selected from hydrogen, halogen, alkyl, alkenyl, hydroxyl,O-alkyl, --S-alkyl, --C(O)-alkyl, and --C(O)-alkenyl, benzyl, benzylcarbamate.

In G₃ and G₄ the R₁₁ and R₁₂ groups are independently selected. For example, in one aspect, in a metal ion chelator of the present invention, the R₁₁ and R₁₂ groups can be the same in G₃ and G₄. In another aspect, at least one of the R₁₁ and R₁₂ groups differs as between G₃ and G₄.

In another aspect of the present disclosure, G₁ can be selected from optionally substituted -C₁₋₈ alkyl, -C₁-₈-alkenyl, aryl, -CH₂(aryl), and -CH(aryl)₂, and heteroaryl.

In another aspect of the present disclosure, when Q is S, G₁ can be selected from optionally substituted substituted -C₁₋₈ alkyl, -C₁₋₈ -alkenyl, aryl, - CH₂(aryl), and -CH(aryl)₂, and heteroaryl.

In another aspect of the present disclosure, when Q is S, G₁ can be selected from NH(C₁₋₆-alkenyl) and --N(aryl)₂.

With reference to an aspect of the disclosure, in a present iron chelator compound, such as a compound represented by formulas 1 through 7, G₃ and G₄ can be the same or different. In one embodiment, G₃ and G₄ are the same. In another embodiment G₃ and G₄ are different.

In regards to the "R" groups in formulas 1, 2 or 3, R₂ may preferably be hydrogen, R₃ may preferably be an alkyl or alkenyl group, R₄ may preferably be hydrogen, and R₁₀, R₂₀ and may preferably be hydrogen, and R₂₀ and may be hydrogen provided G₃ is not alkenyl or alkyl (straight or branched chain).

Chelation refers to the formation or presence of bonds (or other attractive interactions) between two or more separate binding sites within the same ligand and a single central atom. This type of binding of ions suppresses biological activity associated with these ions.

Herein, "(thio)semicarbazone" includes within its scope semicarbazones, thiosemicarbazones, and derivatives or related analogues thereof. Similarly, as used herein, the generic term "(thio)hydrazone" includes within its scope hydrazones, thiohydrazones and derivatives or related analogues thereof.

Herein, "therapeutically effective amount" is intended to include within its meaning a non-toxic but sufficient amount of a compound or composition of the invention to provide the desired therapeutic effect. The exact therapeutically effective amount of the compound or composition will vary according to factors such as the type of disease of the animal, the age, sex, and weight of the animal, mode of administration, and the ability of the compound or composition to permeate cell membranes and chelate metal ions, such as iron, in cells of the animal. Dosage regime can be adjusted to provide the optimum therapeutic response. For example, several divided doses can be administered daily or the dose can be proportionally reduced as indicated by the exigencies of the therapeutic situation.

Herein "treatment," refers to any and all uses which remedy a disease state or symptoms, prevent the establishment of disease, or otherwise prevent, hinder, retard, or reverse the progression of disease or other undesirable symptoms in any way-whatsoever.

The methods described herein advantageously utilize (thio) semicarbazone compounds and (thio) hydrazone compounds as a novel class of anti-HIV-1 agents to treat and inhibit HIV-1 replication, particularly in HIV-1 infected cells.

Cellular CDK2 and CDK9 are important for the regulation of HIV-1 replication and the present ion chelator compounds are molecules that inhibit their HIV-1 transcription associated activity. Additionally, the compounds described above also induce expression of NF-kB inhibitor, IKBα, and HO-1, wherein expression of these genes are known to interfere with the replication of HIV-1. This provides a novel therapeutic method for treating conditions associated with HIV-1 infected cell proliferation.

The (thio) semicarbazone compounds and (thio) hydrazone compounds can act as chelating metal ions, including iron ions and induce IkB and inhibit CDK2, particularly in HIV-1 infected cells to treat and inhibit HIV-1 replication.

In particular, the present disclosure relates to (thio)semicarbazone and (thio)hydrazone compounds modified form of iron chelators comprised of compounds represented by formula 1 and in particular compounds represented by formula 4, preferably representative PpYeT and PpYaT compounds, or a pharmaceutically acceptable salt(s) thereof.

Compounds according to the present disclosure such as represented by formulas 1 through 7, can be readily, suitably prepared.

A compound according to the present invention, and also a compound represented by any of formulas 1 through 7, can be prepared by means of a Schiff base condensation reaction in which a ketone or aldehyde is condensed with either an acid hydrazide or acid thiosemicarbazide of choice to produce the corresponding (thio)semicarbazone or (thio)hydrazone compound having a desired substitution pattern.

Exemplary condensation reactions can be conducted under conditions known to those skilled in the art. For example, suitable solvent systems include acetic acid ethanol, methanol, ethanol/water, methanol/water, or other common organic solvents such as acetone, benzene, toluene, at temperatures of about 20-23°C, etc. A reaction product is a metal ion chelator according to the present invention, or also compounds represented by formulas 1, 2, 3, 4, 5, 6, or 7, and the desired compounds are purified using standard techniques, including recrystallisation from a suitable solvent, and column chromatography.

As an alternative, thiosemicarbazones and thiohydrazones are prepared from the corresponding semicarbazone and hydrazone compounds, respectfully, using methods known to those skilled in the art. For example, a C=O group is converted into a C=S group using Lawesson's reagent under standard conditions. Lawesson's reagent is commercially available. It can also be conveniently prepared in the laboratory by heating a mixture of anisole with phosphorus pentasulfide until the mixture is clear and no more hydrogen sulfide is formed, then recrystallized from toluene or xylene. The main use of Lawesson's reagent is the thionation of carbonyl compounds.

Exemplary compounds according to the present invention can be synthesized by reacting 2-phenyl-1-pyridin-2yl-ethanone with a suitable nitrogen containing compound. For example, suitable nitrogen containing compounds for producing a product in which G₁ represents -NR₂R₃ are represented by the formula: 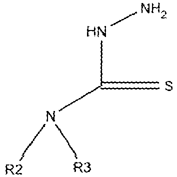 wherein R₂ and R₃ are as described elsewhere herein. In such synthesis, for example, a mixture of reagents can be boiled in a suitable medium, such as a water-ethanol mixture (preferably a 1:1 water:ETOH mixture), the reaction mixture is cooled, and the precipitated product is collected and worked up.

Compounds according to the present disclosure having a G₃ group that is different from the G₄ group can be similarly prepared.

In the course of synthesizing a metal ion chelator herein protecting groups can be used as needed or as appropriate. Protecting groups are described in literature, including Wuts et al., Greene's Protective Groups in Organic Synthesis, John Wiley, (4th ed. 2006), as an example. For instance, various N-protecting groups include benzyl and carbamates, such as t-butylcarbamate and benzylcarbamate.

The present disclosure includes within its scope all isomeric forms of compounds according to the present invention, including those represented by formulas 1, 2, 3, 4, 5, 6, and 7, and metal ion complexes thereof. For example, as appropriate, the present disclosure includes all possible enantiomers, diasteriomers, racemates, cis isomers, trans isomers, (R), (S), (+), (-), .DELTA., and .DELTA. isomers of the compounds and/or their metal complexes.

In some embodiments of the disclosure a composition comprises one or more metal ion complexes of a compound according to the present invention, such as representative PpYeT or PpYaT compounds, or a pharmaceutically acceptable salt(s) thereof. Suitable metal ions include transition metal ions. For example, the metal ions are selected from Periods 4 and 5 of the Periodic Table, such as iron (II), iron (III), copper (II), zinc (II), palladium (II), platinum (II), or gallium (III).

According to another aspect of the invention, a pharmaceutical composition comprises a pharmaceutically acceptable diluent, adjuvant or carrier together with at least one compound according to the present invention, or a pharmaceutically acceptable salt(s) thereof. For example, a pharmaceutical compound comprises at least one of representative PpYeT or PpYaT compounds.

Pharmaceutical compositions according to the invention are formulated for subcutaneous or intravenous injection, oral administration, inhalation, transdermal application, prophylactics (including condoms), or rectal administration. For example in one embodiment of the invention the composition is formulated for intravenous administration. In another embodiment the composition is formulated for oral administration.

Transcription is an essential process for HIV-1 replication. HIV-1 Tat is a small 10 kDa viral protein that activates HIV-1 transcription by binding to the bulge of the transcription activation response (TAR) RNA, an RNA hairpin present at the 5' end of all HIV-1 transcripts. Tat interacts with numerous transcriptional regulatory factors, and presumably by virtue of its interaction with TAR RNA, recruits these factors to the HIV-1 promoter. Tat-recruited factors include RNA polymerase II (RNA pol II) kinase, CDK9/cyclin T1, histone-modifying enzymes and chromatin remodeling complexes (Nekhai, 2006). The HIV-1 promoter located in the 5' long-terminal repeat (LTR) contains binding sites for transcriptional activators and repressor including two NF-kB binding sites. Treatment of the cells with TNFa induces NF-kB and activates basal HIV-1 transcription. The loop of TAR RNA binds cyclin T1, thus promoting CDK9/cyclin T1 recruitment (Wei et al., 1998). CDK9 phosphorylates the C-terminal domain (CTD) of RNA pol II and this phosphorylation promotes elongation of HIV-1 transcription (Isel et al., 1999 and Chen et al., 1999). CDK9/cyclin T1 also phosphorylates human Spt5 subunit of DSIF and the RD subunit of NELF, which may help to relieve the negative effects of DSIF and NELF on transcription elongation (Loyer etal. 2005).

Progression through the G1, S, G2 and M phases of the cell cycle is regulated by sets of CDKs bound to corresponding cyclins (Morgan., 1997). Transition through the G1 phase is regulated by Cdk4/cyclin D1 and by CDK6/cyclin D3. CDK2/cyclin E is active at the late G1 phase and is responsible for G1/S transition. Progression through the S-phase is regulated by CDK2/cyclin A. DNA synthesis during the S-phase is critically relied on the enzymatic activity of ribonucleoside reductase (Tsimberidou et al., 2002). The S/G2 transition is regulated by CDK1/cyclin A. CDK1/cyclin B is responsible for G2/M transitions and for the completion of mitosis. HIV-1 transcription is inducible by Tat only at G1 phase, while at G2 phase it is Tat-independent (Kashanchi et al., 2000). Tat interacts with CDK2/cyclin E (Nekhai 2002). CDK2/cyclin E phosphorylates the CTD of RNA pol II and also phosphorylates Tat *in vitro.* Intracellular Tat is a substrate for CDK2 phosphorylation and that mutation in Tat's Ser16 and Ser 46 residues prevented phosphorylation by CDK2 and inactivated Tat's function in transcription and viral replication (Ammosova et al. 2006

Transcription is an essential process for HIV-1 replication.

HIV-1 Tat is a essential small 10 kDa viral protein that activates HIV-1 transcription. Its binding to regulatory protein domains, such as Tat, CDK2, CDK9/cyclin and NFkB, is metal ion dependent, in particular, iron (II) and iron (III) dependent.

Compounds represented by any of formulas 1, 2, 3, 4, 5, 6, and 7, such as representative PpYeT and/or PpYaT compounds, or pharmaceutically acceptable salts thereof defined herein are useful for modifying protein binding involved in HIV-1 replication, and particularly in HIV-1 transcription, which are sensitive to iron concentration. Examples of such proteins include HIV-1 Tat, TAR RNA and IkB. Administering a therapeutically effective dosage amount of such a compound(s) or a salt(s) thereof modifies expression of these proteins sensitive to iron concentration that are involved in HIV-1 replication.

With reference to another aspect of the disclosure compounds of formulas 1, 2, 3, 4, 5, 6, and/or 7 herein may up-regulate expression of a cellular gene, wherein the upregulated cellular gene acts as a cellular inhibitor of HIV replication, such as IkB gene. Alternatively, the present compounds may down regulate a viral gene, such as a retroviral transcription binding protein involved with binding to transcription activation response (TAR) RNA or other transcription regulatory factors.

According to an aspect of the invention there is provided compounds of the invention for use in a method of inhibiting HIV-1 transcription comprising exposing an infected cell to an effective amount of at least one compound of the invention, such as representative PpYeT and/or PpYaT compounds, or combination thereof as defined herein, or a metal ion complex thereof, or a pharmaceutical composition comprising at least one compound of the invention or a metal ion complex thereof together with a pharmaceutically acceptable diluent, adjuvant, or excipient. A salt from of the compound(s) can be used in the method.

According to another aspect of the disclosure a method of HIV-1 inhibition therapy in a mammal in need of treatment comprises administering to the mammal a therapeutically effective amount of (a) at least one compound represented by any of formulas 1 through 7, such as formulas 1 or 4, which representative PpYeT and/or PpYaT compounds, or (b) a pharmaceutical composition comprising at least one compound of represented by any of formulas 1 through 7, such as formulas 1 or 4, which includes representative PpYeT and/or PpYaT compounds, together with a pharmaceutically acceptable diluent, adjuvant, or excipient. In a further aspect of the disclosure, the mammalian cytotoxicity at HIV-1 treatment concentrations is improved when compared to conventional methods of HIV-1 treatment. Improved mammalian cytotoxicity includes no or at least reduced mammalian cytotoxicity.

The term mammal includes human, non-human primate, murine, bovine, ovine, equine, caprine, leporine, avian, feline, porcine, or canine. By preference, in a treatment embodiment mammal includes human.

An embodiment of the disclosure is directed to methods for the use of at least one compound represented by any of formulas 1 through 7, such as formula 1 or 4, which includes representative PpYeT and/or PpYaT compounds, as defined herein, or a metal ion complex thereof, for the preparation of a medicament for treating HIV-1 infections or associated disorders in mammals.

With reference to an embodiment of the invention, at least one compound according to the present invention, such as representative PpYeT or PpYaT compounds, or a composition comprising the compound(s), or a pharmaceutically acceptable salt of the compound(s) can be used to inhibit HIV-1 replication *in vitro* cellular proliferation.

In an aspect of the novel method of treatment, when used for the treatment of HIV-1, a compound according to the present invention, which includes representative PpYeT or PpYaT compounds, may be administered alone. Alternatively, the compounds are administered as a pharmaceutical formulation which comprises at least one compound according to the present invention, which includes representative PpYeT or PpYaT compounds, a metal ion complex thereof, or a pharmaceutically acceptable salt. It will be appreciated that the pharmaceutical dosing can include more than one compound according to the present invention and/ or a pharmaceutically acceptable salt thereof.

Suitable pharmaceutically acceptable salts of the compounds of the present invention therefore include acid addition salts. Suitable pharmaceutically acceptable acid addition salts of compounds according to the present invention, which includes salts of representative PpYeT or PpYaT compounds, may be prepared by mixing a pharmaceutically acceptable acid such as hydrochloric acid, sulfuric acid, methanesulfonic acid, succinic acid, fumaric acid, maleic acid, benzoic acid, phosphoric acid, acetic acid, oxalic acid, carbonic acid, tartaric acid, or citric acid with a compound according to the present invention. The pharmaceutical composition includes a dispersion of at least one compound according to the present invention, such as representative PpYeT or PpYaT compounds, in a dispersion medium. For example, a dispersion medium can comprise glycerol, liquid polyethylene glycols, and mixtures thereof and in oils.

A pharmaceutical preparation or composition can contain a preservative to prevent the growth of microorganisms as may be customary under ordinary conditions of storage and use.

Pharmaceutical compositions suitable for injection include sterile aqueous solutions or dispersions and sterile powders for the extemporaneous preparation of sterile injectable solutions or dispersions. A composition is stable under the conditions of manufacture and storage and optionally includes a preservative to stabilize the composition against the contaminating action of microorganisms such as bacteria and fungi.

A pharmaceutical composition further may include a suitable buffer to minimize acid hydrolysis. Suitable buffer agents are well known to those skilled in the art and include, but are not limited to, phosphates, citrates, carbonates and mixtures thereof.

Generally, an effective dosage per 24 hours may be in the range of about 0.0001 mg to about 1000 mg per kg body weight; suitably, about 0.001 mg to about 750 mg per kg body weight; about 0.01 mg to about 500 mg per kg body weight; about 0.1 mg to about 500 mg per kg body weight; about 0.1 mg to about 250 mg per kg body weight; or about is 1.0 mg to about 250 mg per kg body weight. Still suitably, an effective dosage per 24 hours may be in the range of about 1.0 mg to about 200 mg per kg body weight; about 1.0 mg to about 100 mg per kg body weight; about 1.0 mg to about 50 mg per kg body weight; about 1.0 mg to about 25 mg per kg body weight; about 5.0 mg to about 50 mg per kg body weight; about 5.0 mg to about 20 mg per kg body weight; or about 5.0 mg to about 15 mg per kg body weight.

By way of example, suitable dosage forms in accordance with the present invention include the following: TABLE-US-00001 Tablet Compound of formula 1 and/or 4 0.01 to 20 mg, generally 0.1 to 10 mg, starch 10 to 20 mg, lactose 100 to 250 mg, gelatin 0 to 5 mg, magnesium stearate 0 to 5 mg; an injectable solution containing a compound of formula 1 and/or 4 and 4 0.01 to 20 mg, generally 0.1 to 10 mg, sodium chloride 8.5 mg, potassium chloride 3 mg, calcium chloride 4.8 mg, sterile water for injection, q.s. to 10 ml.

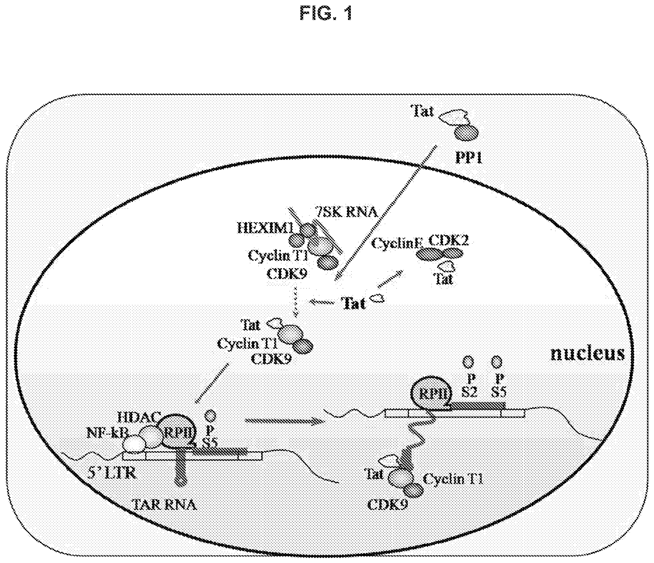

Figure. 1 is an illustration of activation of HIV-1 transcription by Tat. Association of Tat's Q35VCF38 sequence with PP1's RVxF-accommodating cleft helps Tat to bind PP1 and translocates it to the nucleus. The binding of Tat and PP1 is required for nuclear translocation. Nuclear PP1 directly or indirectly facilitates dephosphorylation of CDK9 on Thr186. Dephosphorylated CDK9 in complex with cyclin T1 dissociates from 7SK RNA and HEXIM1 protein and CDK9 undergoes phosphorylation on Thr186 to gain enzymatic activity. Tat recruits reactivated CDK9/cyclin T1 to HIV-1 TAR RNA where CDK9 phosphorylates RNA polymerase II C-terminal domain (CTD) and induces HIV-1 transcription. Tat interacts with CDK2/cyclin E and undergoes phosphorylation on Ser16 and Ser46 residues. Without limiting the inventions herein, the molecular mechanism for preventing HIV-1 transcription by the present chelators is believed to involve their targeting and resulting inhibition of CDK9/cyclin T1 activity in addition to CDK2 activity. For example, during viral entry, HIV-1 replication is dependent on the activity of host cell ribonucleotide reductase that contains non-heme iron, which is important for enzymatic activity (Tsimberidou et al. 2002).

The inventions are further described in the following non-limiting Examples.

### EXAMPLES

### Synthesis of PpY (2-phenyl-1-pyridin-2yl-ethanone)

The general steps in an exemplary synthesis of PPY are depicted in the following reaction scheme.

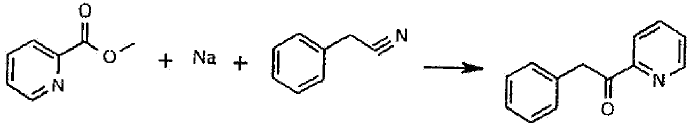

A solution of the nitrile and the ester in about 100ml of THF are added dropwise into suspension of NaH in about 300ml THF while stirring and cooling in a water bath. The reagents are mixed together and the water bath is removed. After stirring overnight and evaporating the solvent, the residue is dissolved in a minimal amount of water and extracted with 2 x 200ml of chloroform. The water layer is acidified with about 300ml of concentrated HCl and is boiled under reflux for an appropriate sufficient time, which can be up to about a day. The reaction mixture is left standing. A hydrochloride precipitate forms, is filtered, is washed with acetone and is air-dried, with a yield of 16.0 g.

About 13.7g of the hydrochloride salt is dissolved in about 100ml of methanol. Sodium hydrocarbonate is added portionally under stirring conditions until gas stopped evolving and is followed by about 30 min of stirring. Then the solution is filtered and evaporated, the residue is treated with about 50ml of water and about 100ml of chloroform. The organic layer is removed, and the water layer is extracted with about 100ml of chloroform.

The organic layer is dried with sulphate and evaporated. The residue is left to crystallize. After crystallization the product is washed with a small amount of cold hexane and air-dried, with a yield of 9.8 g (84%).

The aqueous solution is extracted with 3 x 200ml of chloroform, and then sodium hydrocarbonate is added to increase pH to about 7-8. The solution is extracted with 3 x 300ml of chloroform. The organic layer is dried with sulphate and evaporated. The residue is treated with about 100ml of hot hexane, which is later separated from oil. After the solution is allowed to cool, it is decanted and evaporated. The residue is treated with about 20 ml of hexane and left in a cool place for crystallization. The precipitated product is filtered and is washed with a small amount of cold hexane, with a ketone yield of 2.9 g.

It will be appreciated that different ester and nitrile combinations can be selected and reacted by adapting this synthesis, with the resultant products being allowed to react with a suitable nitrogen containing compound to synthesize other metal ion chelators according to the present invention.

### Synthesis of PpYeT compound

The general steps in a synthesis of the PpYeT compound are depicted in the following reaction scheme:

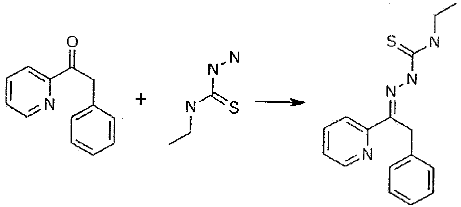

A mixture of the reagents is boiled in about 20 ml of a water-ethanol mixture of about (1:1) for about 24 hours. After it is allowed to cool. A precipitated product is filtered and dried under vacuum at about 60°C.

It will be appreciated that other compounds according to the present invention, can be prepared by selecting the compound to react with PpY and adapting this synthesis.

### Synthesis of PpYaT compound

The general steps in a synthesis of the PpYaT compound are depicted in the following reaction scheme:

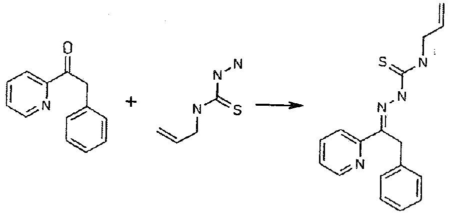

A mixture of the reactants are boiled in about 20 ml of a water-ethanol mixture of about (1:1) for about 24 hours. After it is allowed to cool. A precipitated product is filtered and dried under vacuum at about 60°C.

Selecting other nitrogen containing reactants to react with PPY can produce other compounds according to the present invention. For example, it will be appreciated that other compounds according to the present invention, can be prepared by selecting a different nitrogen compound and adapting this synthesis.

### EXAMPLE 1

### Effect of iron chelators on one round of HIV-1-Luc replication in CEM cells.

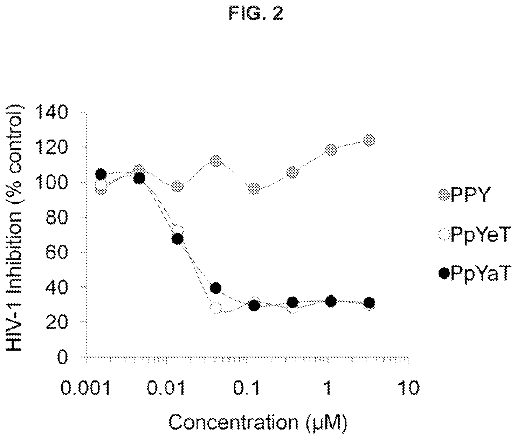

As shown in Figure 2, human CEM cells were infected with VSVG-pseudotyped pNL4-3.Luc.R-E- (HIV-1 Luc) virus for about 18 h at 37°C and then treated for about 24 h at about 37°C with the indicated concentrations of iron chelators. Luciferase activity was then measured. Both PpYeT and PpY-aT inhibit HIV-1 replication at the nanomolar (IC₅₀ = 14nM) concentrations, comparable to the effect of previously studied Bp4-eT chelator (IC₅₀ = 7.5nM).

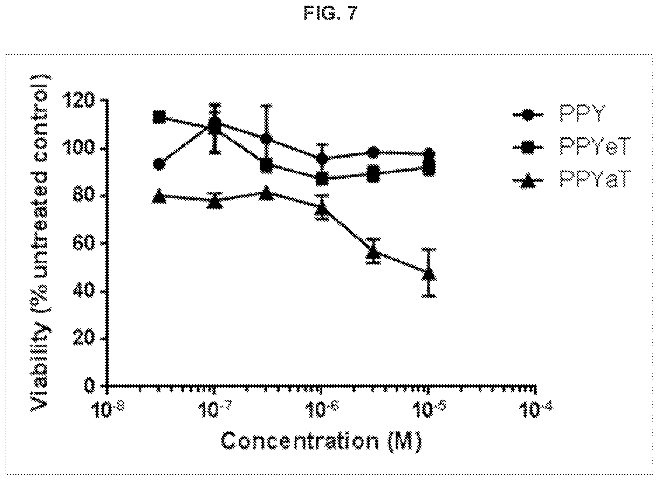

Figure 7 further shows the toxicity of iron chelators under the same HIV-1 infection conditions disclosed in the above paragraph, and then treated for about 72 h at about 37°C with the indicated concentrations of the named iron chelators. Luciferase activity was then measured.

Bp4-eT and Bp4-aT are represented by the following formulas:

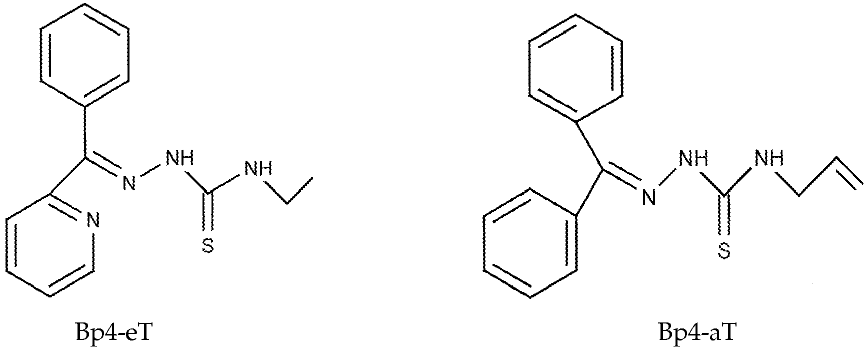

Structure-activity relationships of the PpYeT and the previously described Bp4-eT and Bp4-aT compound indicate that even though these classes of compound appear to have a similar metal-binding site, the lipophilicity, efficacy of metal ion chelation, and antiviral properties of the respective classes differ substantially and are unpredictable. Subtle differences of the compounds in the binding motif of the targeted domain of the HIV-1 associtated protein may account for these results. This supports the aspect of the present disclosure that involves the use of a mixture of inhibitors that include at least one of our iron chelators because inhibitors can affect slightly different binding motifs of the PP1 domain and resistance to inhibitors having different binding motifs would be more difficult. This can also address the fact that HIV-1 replication may be error prone.

### EXAMPLE 2

### Toxicity of iron chelators in CEM cells.

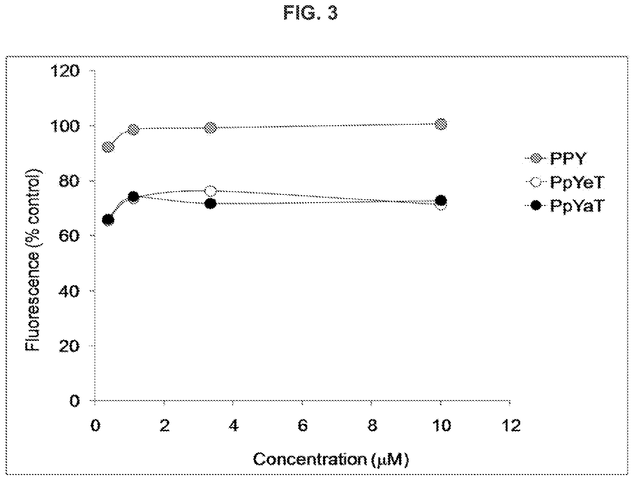

Human CEM cells (derived from T cells) were treated with the indicated concentrations of iron chelators for about 24 h at about 37°C as shown in Figure. 3. Cells were treated with 0.4 mM Calcein-AM for 10 min and calcein fluorescence was measured at 485 nm excitation and 515 nm emission on Spectrofluorimeter equipped with the robotic arm. Neither PPY, nor representative PpYeT and PpYaT compounds show significant toxicity, at micromolar concentrations. However, the relative lack of toxicity of the compounds of the present invention, such as representative PpYeT or PpYaT compounds, combined with the high specificity of these compounds make them well suited for inhibition of HIV-1 progression *in vivo.* The emergence of drug-resistant HIV-1 strains can be delayed or even prevented, if from the beginning, the inhibitors / drugs are administered at levels higher than the EC₅₀. Nontoxic micromolar concentrations that are only 100 to 250-fold higher than the EC₅₀, completely suppressed virus breakthrough and cleared the cells of HIV-1 as assessed by the absence of proviral DNA in the cellular genome. The term half maximal effective concentration (EC₅₀) refers to the concentration of a drug which induces a response halfway between the baseline and maximum after some specified exposure time *in vivo.* It is commonly used as a measure of drug's potency.

### EXAMPLE 3

### Iron chelators efficiently chelate cellular iron

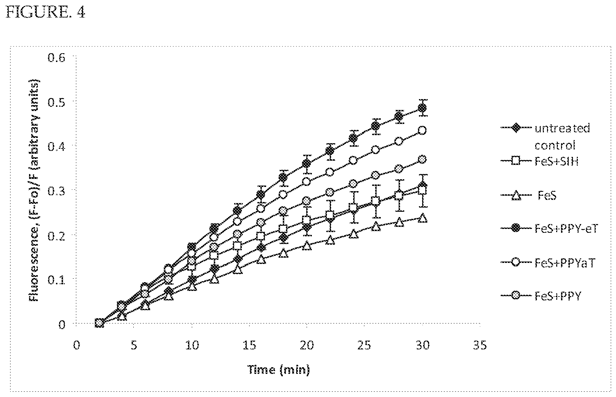

To assess iron chelating efficiency, human acute monocytic leukemia cell line (THP-1) cells were untreated (control) or treated with FeS for about 1 hr at about 37°C, and then loaded with iron sensor, calcein-AM for about 10 min at about 37°C. After washing with phosphate buffer solution (PBS), cells were treated with various iron chelators. In Figure. 4, calcein fluorescence was measured in a real-time PCR machine as a function of time. Fractional fluorescence (F-Fi)/Fi is proportional to the amount of chelatable iron. PPY-eT and PPY-aT are better than the control SIH compound.

### EXAMPLE 4

### Iron chelators induce expression of IkB gene.

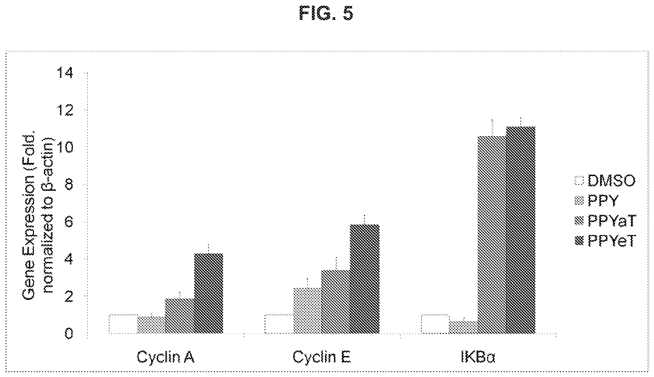

To assess inducing expression of the IkB gene with the present iron chelators, human 293T cells were treated with vehicle (DMSO), PPY control compound and iron chelators, representative PpYeT and PpYaT compounds. After about 24 hours of treatment, RNA was extracted, reverse transcribed and analyzed on a real-time PCR machine. Generic primers for β-actin were used for reference. The DDCt analysis (delta-delta-Ct or ΔΔCt algorithm, is a convenient method to analyze the relative changes in gene expression), was performed using DMSO-treated sample as a reference target and reference control. Exemplary iron chelator compounds, such as representative PpYeT and PpYaT compounds, induce expression of IkB, which is consistent with a mechanism for cyclin E deregulation and CDK2 inhibition (see Figure. 5). As shown in Figure 1, CDK2 regulates HIV-1 transcription and include (1) phosphorylation of Tat by CDK2 that enhance interaction of P-Tat with cyclin T1 or histone acetyltransferases and recruitment of co-activators to HIV-1 transcription complex, or ubiquitination of P-Tat that enhance its transcriptional activity or its stability; and (2) CDK2/cyclin E-mediated phosphorylation of RNAPII CTD heptapeptide repeats. This example demonstrates the importance of the interaction of the Tat protein on multiple levels. This suggests an unexpected improved efficacy for compounds of the present invention, including the pepresentative PPYeT and PPYaT compounds involved in modifying HIV-1 transcription on multiple levels.

### EXAMPLE 5

### Iron chelators induce expression of heme oxygenase-1.

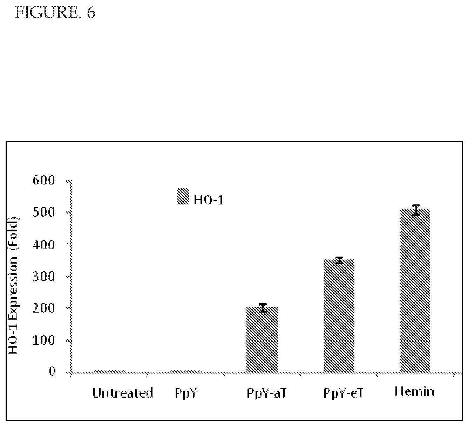

To assess inducing expression of the heme oxygenase-1 gene with the present iron chelators, human promonocytic THP-1 cells were untreated or treated with PPY as a control compound, iron chelators (PPYeT and PPYaT) and as a positive control, 100 µM hemin. After about 24 hours of treatment, RNA was extracted, reverse transcribed and analyzed on a real-time PCR machine. Generic primers for β-actin were used for reference. The DDCt analysis (delta-delta-Ct or ΔΔCt algorithm, is a convenient method to analyze the relative changes in gene expression), was performed using DMSO-treated sample as a reference target and reference control. Exemplary iron chelator compounds, such as representative PpYeT and PpYaT compounds, induce expression of heme oxygenase-1 (HO-1) (see Figure. 6). Treatment with hemin as control also induced expression of HO-1. The expression of HO-1 has been previously shown to inhibit HIV-1 in macrophages and T-cells treated *in vitro* with hemin, an analog of heme, Devadas et al (2006 &, 2010) and *in vivo* in HIV-1 infected humanized mice treated with hemin Devadas et al (2006). This suggests an unexpected improved efficacy for PPYeT and PPYaT involved in induction of HIV-1-restriciting HO-1. Accordingly, this Example demonstrates that the present iron chelators of the present invention can induce the expression of HO-1 to thereby inhibit or at least retard HIV-1 replication.

### EXAMPLE 6

### Effect of iron chelators on one round of HIV-1-Luc replication in CEM cells.

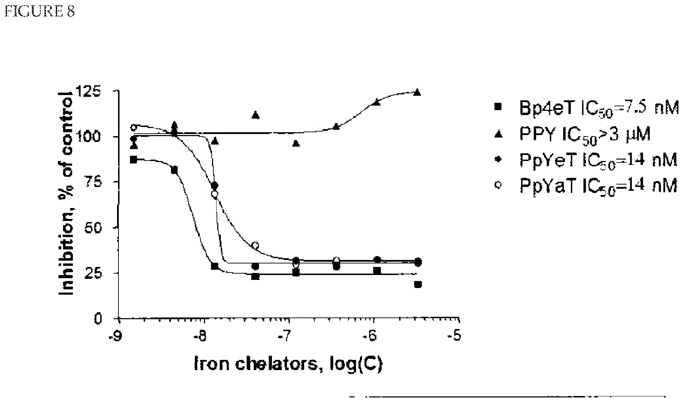

CEM cells were infected with VSVG-pseudotyped pNL4-3. Luc.R-E-HIV-1 Luc) virus for 18 h at 37°C and then treated for 24 h at 37 °C with the indicated concentrations of iron chelators as shown in Figure 8. Luciferase activity was then measured. Data were analyzed in Prizm Software. Both of these representative compounds PpYeT and PpYaT, inhibit HIV-1 replication comparable to the effect of previously studied Bp4eT chelator. Advantageously compounds of the present invention are less toxic than Bp4eT and Bp4aT as seen from other data for representative compounds.

The following references are cited:
1. Drakesmith, H. and A. Prentice, Viral infection and iron metabolism. Nat Rev Microbiol, 2008. 6(7): p. 541-52.
2. Porter, J.B., Deferasirox: An effective once-daily orally active iron chelator. Drugs Today (Barc), 2006. 42(10): p. 623-37.
3. Yuan, J., D.B. Lovejoy, and D.R. Richardson, Novel di-2-pyridyl-derived iron chelators with marked and selective antitumor activity: in vitro and in vivo assessment. Blood, 2004.104(5): p. 1450-8.
4. Whitnall, M., et al., A class of iron chelators with a wide spectrum of potent antitumor activity that overcomes resistance to chemotherapeutics. Proc Natl Acad Sci U S A, 2006.103(40): p. 14901-6.
5. Nekhai, S. and K.T. Jeang, Transcriptional and post-transcriptional regulation of HIV-1 gene expression: role of cellular factors for Tat and Rev. Future Microbiol, 2006. 1: p. 417-26.
6. Wei, P., et al., A novel CDK9-associated C-type cyclin interacts directly with HIV-1 Tat and mediates its high-affinity, loop-specific binding to TAR RNA. Cell, 1998. 92(4): p. 451-62.
7. Isel, C. and J. Karn, Direct evidence that HIV-1 Tat stimulates RNA polymerase II carboxyl-terminal domain hyperphosphorylation during transcriptional elongation. J Mol Biol, 1999. 290(5): p. 929-41.
8. Chen, D. and Q. Zhou, Tat activates human immunodeficiency virus type 1 transcriptional elongation independent of TFIIH kinase. Mol Cell Biol, 1999. 19(4): p. 2863-71.
9. Loyer, P., et al., Role of CDK/cyclin complexes in transcription and RNA splicing. Cell Signal, 2005.17(9): p. 1033-51.
10. Prelich, G., RNA polymerase II carboxy-terminal domain kinases: emerging clues to their function. Eukaryot Cell, 2002. 1(2): p. 153-62.
11. Fujinaga, K., et al., Dynamics of human immunodeficiency virus transcription: P-TEFb phosphorylates RD and dissociates negative effectors from the transactivation response element. Mol Cell Biol, 2004. 24(2): p. 787-95.
12. Morgan, D.O., Cyclin-dependent kinases: engines, clocks, and microprocessors. Annu Rev Cell Dev Biol, 1997.13: p. 261-91.
13. Tsimberidou, A.M., Y. Alvarado, and F.J. Giles, Evolving role of ribonucleoside reductase inhibitors in hematologic malignancies. Expert Rev Anticancer Ther, 2002. 2(4): p. 437-48.
14. Kashanchi, F., et al., Cell cycle-regulated transcription by the human immunodeficiency virus type 1 Tat transactivator. J Virol, 2000. 74(2): p. 652-60.
15. Nekhai, S., et al., Cell cycle-dependent stimulation of the HIV-1 promoter by Tat-associated CAK activator. Virology, 2000. 266(2): p. 246-56.
16. Nekhai, S., et al., HIV-1 Tat-associated RNA polymerase C-terminal domain kinase, CDK2, phosphorylates CDK7 and stimulates Tat-mediated transcription. Biochem J, 2002. 364(Pt 3): p. 649-57.
17. Deng, L., et al., HIV-1 Tat interaction with RNA polymerase II C-terminal domain (CTD) and a dynamic association with CDK2 induce CTD phosphorylation and transcription from HIV-1 promoter. J Biol Chem, 2002. 277(37): p. 33922-9.
18. Ammosova, T., et al., Phosphorylation of HIV-1 Tat by CDK2 in HIV-1 transcription. Retrovirology, 2006. 3: p. 78.
19. Ammosova, T., et al., Dephosphorylation of CDK9 by protein phosphatase 2A and protein phosphatase-1 in Tat-activated HIV-1 transcription. Retrovirology, 2005. 2: p. 47.
20. Debebe, Z., et al., Iron chelators of the di-2-pyridylketone thiosemicarbazone and 2-benzoylpyridine thiosemicarbazone series inhibit HIV-1 transcription: identification of novel cellular targets--iron, cyclin-dependent kinase (CDK) 2, and CDK9. Mol Pharmacol, 2011. 79(1): p. 185-96.
21. Devadas, K. and S. Dhawan, Hemin activation ameliorates HIV-1 infection via heme oxygenase-1 induction. J Immunol, 2006.176(7): p. 4252-7.
22. Devadas, K., I.K. Hewlett, and S. Dhawan, Lipopolysaccharide suppresses HIV-1 replication in human monocytes by protein kinase C-dependent heme oxygenase-1 induction. J Leukoc Biol, 2010. 87(5): p. 915-24.

While preferred embodiments have been described in detail, variations and modifications can be effected within the scope of the presented embodiments.

## Claims

1. A compound of a formula: 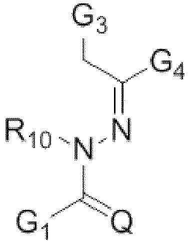 wherein
Q is O or S;
G₁ is NHR₂;
R₂ is selected from an alkyl group, or an alkenyl group;
R₁₀ is hydrogen;
G₃ is selected from an unsubstituted aromatic ring, such as an unsubstituted 5- or 6-membered aromatic ring, or an unsubstituted heteroaromatic ring, such as a 5- or 6-membered heteroaromatic ring;
G₄ is an unsubstituted N-heteroaryl group linked at the 2-position to the core structure of the compound, or
a pharmaceutically acceptable salt of said compound.

2. A compound according to claim 1 represented by the formula: 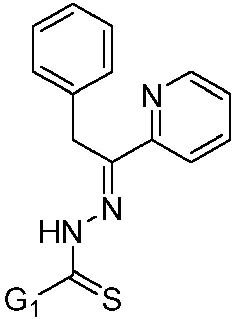 wherein
G₁ is NHR₂; and
R₂ is selected from an alkyl group, or an alkenyl group, or
a pharmaceutically acceptable salt of said compound.

3. A compound according to claim 1 or claim 2, wherein R₂ is selected from an alkyl group having from 1 to 10 carbon atoms, or an alkenyl group having from 2 to 8 carbon atoms.

4. A compound represented by the formula: 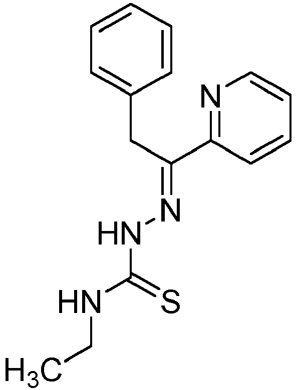 or a pharmaceutically acceptable salt of said compound.

5. A compound represented by the formula: 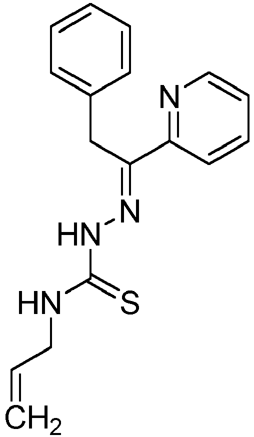 or a pharmaceutically acceptable salt of said compound.

6. The compound of any one of claims 1, 2, 3, 4, or 5, wherein said compound is a pharmaceutically acceptable salt of said compound.

7. A pharmaceutical composition comprising a compound according to any one of claims 1, 2, 3, 4, 5 or 6; and at least one pharmaceutically acceptable excipient or diluent.

8. A compound of any of claims 1, 2, 3, 4, 5 or 6, or a composition according to claim 7, for use in a method for treating a subject infected with or at risk of infection with HIV-1, which method comprises administering to said subject in need of such treatment a therapeutically effective amount of said compound or composition.

9. An *in vitro* method of inhibiting replication of HIV-1 virus, comprising contacting the HIV-1 virus or a cell containing the HIV-1 virus with a compound according to any of claims 1, 2, 3, 4, 5 or 6 or a composition according to claim 7.

10. A compound according to any of claims 1, 2, 3, 4, 5 or 6, or a composition according to claim 7, for use in a method of inhibiting replication of HIV-1 virus, which method comprises contacting the HIV-1 virus or a cell containing the HIV-1 virus with said compound or composition.

11. A compound according to any one of claims 1, 2, 3, 4, 5 or 6, or a composition according to claim 7, for use in a method for treatment of the human body by therapy.

12. A compound for use according to claim 11, wherein the therapy comprises metal ion chelation therapy.

13. A compound for use according to claim 11 or claim 12, wherein the therapy comprises iron chelation therapy.

## Patentansprüche

1. Verbindung der Formel: 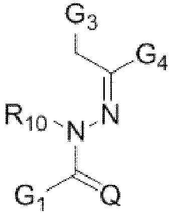 worin
Q für O oder S steht,
G₁ für NHR₂ steht,
R₂ ausgewählt ist aus einer Alkylgruppe oder einer Alkenylgruppe,
R₁₀ für Wasserstoff steht,
G₃ ausgewählt ist aus einem unsubstituierten aromatischen Ring, wie einem unsubstituierten 5- oder 6-gliedrigen aromatischen Ring, oder einem unsubstituierten heteroaromatischen Ring, wie einem 5- oder 6-gliedrigen heteroaromatischen Ring,
G₄ eine unsubstituierte N-Heteroarylgruppe ist, die an der 2-Position mit der Kernstruktur der Verbindung verbunden ist, oder
ein pharmazeutisch annehmbares Salz der Verbindung.

2. Verbindung nach Anspruch 1, dargestellt durch die Formel: 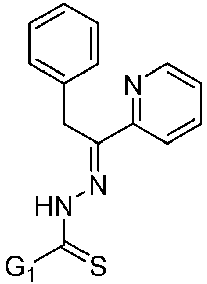 worin
G₁ für NHR₂ steht, und
R₂ ausgewählt ist aus einer Alkylgruppe oder einer Alkenylgruppe oder einem pharmazeutisch annehmbaren Salz der Verbindung.

3. Verbindung nach Anspruch 1 oder Anspruch 2, worin R₂ ausgewählt ist aus einer Alkylgruppe mit 1 bis 10 Kohlenstoffatomen oder einer Alkenylgruppe mit 2 bis 8 Kohlenstoffatomen.

4. Verbindung nach Anspruch 1, dargestellt durch die Formel: 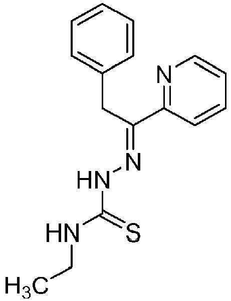 oder ein pharmazeutisch annehmbares Salz davon.

5. Verbindung nach Anspruch 1, dargestellt durch die Formel: 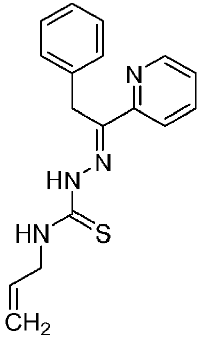 oder ein pharmazeutisch annehmbares Salz davon.

6. Verbindung nach einem der Ansprüche 1, 2, 3, 4 oder 5, worin die Verbindung ein pharmazeutisch annehmbares Salz der Verbindung ist.

7. Pharmazeutische Zusammensetzung, umfassend eine Verbindung nach einem der Ansprüche 1, 2, 3, 4, 5 oder 6 und mindestens einen pharmazeutisch annehmbaren Arzneimittelträger oder ein pharmazeutisch annehmbares Verdünnungsmittel.

8. Verbindung nach einem der Ansprüche 1, 2, 3, 4, 5 oder 6 oder Zusammensetzung nach Anspruch 7 zur Verwendung in einem Verfahren zur Behandlung eines Subjekts, welches mit HIV-1 infiziert ist oder für welches die Gefahr einer HIV-1-Infektion besteht, worin das Verfahren die Verabreichung einer therapeutisch wirksamen Menge der Verbindung oder Zusammensetzung an das einer solchen Behandlung bedürftige Subjekt umfasst.

9. *In vitro*-Verfahren zur Hemmung der Replikation des HIV-1-Virus, umfassend: Inkontaktbringen des HIV-1-Virus oder einer das HIV-1-Virus enthaltenden Zelle mit einer Verbindung nach einem der Ansprüche 1, 2, 3, 4, 5 oder 6 oder einer Zusammensetzung nach Anspruch 7.

10. Verbindung nach einem der Ansprüche 1, 2, 3, 4, 5 oder 6 oder Zusammensetzung nach Anspruch 7 zur Verwendung in einem Verfahren zur Hemmung der Replikation des HIV-1-Virus, worin das Verfahren das Inkontaktbringen des HIV-1-Virus oder einer das HIV-1-Virus enthaltenden Zelle mit der Verbindung oder Zusammensetzung umfasst.

11. Verbindung nach einem der Ansprüche 1, 2, 3, 4, 5 oder 6 oder Zusammensetzung nach Anspruch 7 zur Verwendung in einem Verfahren zur Behandlung des menschlichen Körpers mittels einer Therapie.

12. Verbindung zur Verwendung nach Anspruch 11, worin die Therapie eine Metallion-Chelatisierungs-Therapie umfasst.

13. Verbindung zur Verwendung nach Anspruch 11 oder Anspruch 12, worin die Therapie eine Eisen-Chelatisierungs-Therapie umfasst.

## Revendications

1. Composé de formule : 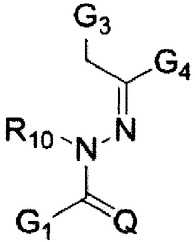 où
Q est O ou S ;
G₁ est NHR₂ ;
R₂ est choisi parmi un groupe alkyle et un groupe alcényle ;
R₁₀ est l'hydrogène ;
G₃ est choisi parmi un cycle aromatique non substitué, comme un cycle aromatique à 5 ou 6 chaînons non substitué, et un cycle hétéroaromatique non substitué, comme un cycle hétéroaromatique à 5 ou 6 chaînons ;
G₄ est un groupe N-hétéroaryle non substitué lié à la position 2 à la structure centrale du composé, ou
sel pharmaceutiquement acceptable dudit composé.

2. Composé selon la revendication 1 représenté par la formule : 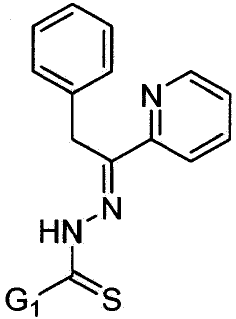 où
G₁ est NHR₂ ; et
R₂ est choisi parmi un groupe alkyle et un groupe alcényle, ou
sel pharmaceutiquement acceptable dudit composé.

3. Composé selon la revendication 1 ou la revendication 2, où R₂ est choisi parmi un groupe alkyle ayant de 1 à 10 atomes de carbone, et un groupe alcényle ayant de 2 à 8 atomes de carbone.

4. Composé selon la revendication 1 représenté par la formule : 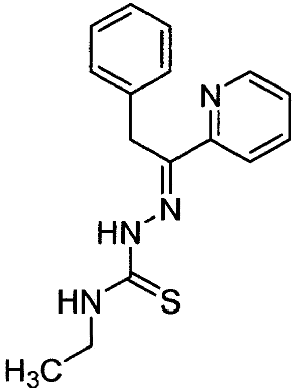 ou sel pharmaceutiquement acceptable dudit composé.

5. Composé selon la revendication 1 représenté par la formule : 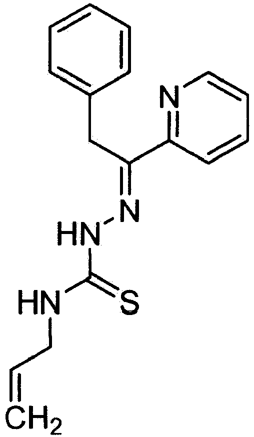 ou sel pharmaceutiquement acceptable dudit composé.

6. Composé selon l'une quelconque des revendications 1, 2, 3, 4 et 5, où ledit composé est un sel pharmaceutiquement acceptable dudit composé.

7. Composition pharmaceutique comprenant un composé selon l'une quelconque des revendications 1, 2, 3, 4, 5 et 6 ; et au moins un excipient ou diluant pharmaceutiquement acceptable.

8. Composé selon l'une quelconque des revendications 1, 2, 3, 4, 5 et 6, ou composition selon la revendication 7, destiné à être utilisé dans un procédé pour traiter un sujet infecté avec ou présentant un risque d'infection avec VIH-1, lequel procédé comprend l'administration audit sujet nécessitant un tel traitement d'une quantité thérapeutiquement efficace dudit composé ou de ladite composition.

9. Procédé *in vitro* d'inhibition de la réplication du virus VIH-1, comprenant la mise en contact du virus VIH-1 ou d'une cellule contenant le virus VIH-1 avec un composé selon l'une quelconque des revendications 1, 2, 3, 4, 5 et 6 ou une composition selon la revendication 7.

10. Composé selon l'une quelconque des revendications 1, 2, 3, 4, 5 et 6, ou composition selon la revendication 7, destiné à être utilisé dans un procédé d'inhibition de la réplication du virus VIH-1, lequel procédé comprend la mise en contact du virus VI-1 ou d'une cellule contenant le virus VIH-1 avec ledit composé ou ladite composition.

11. Composé selon l'une quelconque des revendications 1, 2, 3, 4, 5 et 6, ou composition selon la revendication 7, destiné à être utilisé dans un procédé pour le traitement du corps humain par une thérapie.

12. Composé destiné à être utilisé selon la revendication 11, où la thérapie comprend une thérapie par chélation d'ions métalliques.

13. Composé destiné à être utilisé selon la revendication 11 ou la revendication 12, où la thérapie comprend une thérapie par chélation du fer.
